# EUROPEAN PATENT APPLICATION

(11) **EP 3 437 606 A1**
(43) Date of publication of application: **06.02.2019**
(21) Application number: 18163814.9
(22) Date of filing: 24.03.2018
(51) Int. Cl.: A61F 13/505, A61F 13/64, A61F 13/66, A61F 13/62

(54) **DISPOSABLE DIAPER WITH REUSABLE WAISTBAND**

(30) Priority: 31.07.2017 CN 201710640683
(71) Applicant: Guangzhou Youhu Commodity Co., Ltd., Renhe Town, Baiyun District Guangzhou City Guangdong (CN)
(72) Inventor: PENG, Liang-Lin, Guangzhou City, Guangdong (CN)
(74) Representative: Lang, Christian

(57) **Abstract**

The present invention discloses a disposable diaper with a reusable waistband (including disposable diapers for adults and babies), which comprises a disposable diaper and an attachable waistband. Two waistband attachments are symmetrically formed in the back of the disposable diaper. The attachable waistband is made of a non-woven fabric. Several elastic bands are fixed onto the inner side of the attachable waistband. Two removable adhesive strips are formed in the middle region of the inner side of the attachable waistband and corresponding to the waistband attachments of the disposable diaper. Two corresponding hook-and-loop fasteners are respectively formed on two ends of the attachable waistband. The disposable diaper of the present invention is easy to wear. The attachable waistband of the present invention can be repeatedly used. Therefore, the present invention saves user much money and favors environmental protection.

## Description

This application claims priority for China patent application no. 201710640683.0 filed on July 31, 2017, the content of which is incorporated by reference in its entirety.

### Field of the Invention

The present invention relates to an article for daily use, particularly to a disposable diaper with a reusable waistband.

### Description of the Related Art

The traditional pant-type disposable diaper is very inconvenient for the nursing personnel because it must be taken off completely before replacing a new one. The traditional pad-type disposable diaper is likely to displace from the original position and irritate the user because it lacks a fixing mechanism. The traditional pant-type disposable diaper is abandoned after a single use. The waistband of the traditional pant-type disposable diaper has higher material cost and higher fabrication cost. The use of the traditional pant-type disposable diaper is not only unfavorable for environmental protection but also uneconomical for users.

### SUMMARY OF THE INVENTION

In order to solve the abovementioned problems, the present invention provides a disposable diaper with a reusable waistband, which is easy to wear, economical for users, and favorable for environmental protection because the waistband is reusable.

In order to achieve the abovementioned objectives, the present invention proposes a disposable diaper with a reusable waistband, which comprises a disposable diaper and an attachable waistband. Two waistband attachments are symmetrically formed in the back of the disposable diaper. The attachable waistband is made of a non-woven fabric. Several elastic bands are fixed onto the inner side of the attachable waistband. Two removable adhesive strips are formed in the middle region of the inner side of the attachable waistband and corresponding to the waistband attachments of the disposable diaper. Two corresponding hook-and-loop fasteners are respectively formed on two ends of the attachable waistband.

In one embodiment, the girth of the disposable diaper for adults has three sizes: 155-165cm for a large size, 165-175cm for an enlarged size, and 175-190cm for an extra-large size; the girth of the disposable diaper for babies has three sizes: 60-80cm for a large size, 80-100cm for an enlarged size, and 95-115cm for an extra-large size.

In one embodiment, the hook-and-loop fastener is 30-50cm in length and 20-35cm in width.

In one embodiment, the attachable waistband has 6-18 pieces of elastic bands, which are fixed onto the inner side of the attachable waistband with an adhesive glue.

In one embodiment, the attachable waistband is 20-80cm in width.

In one embodiment, the disposable diaper includes an external water-proof layer, a core layer and an inner cotton layer. The core layer is interposed between the external water-proof layer and the inner cotton layer and disposed between the two waistband attachments.

In one embodiment, the core layer is made of wood pulp and a polymer material.

The present invention has the following advantages:
1. The present invention is easy to wear: the present invention not only overcome the disadvantage of the traditional pad-type disposable diaper, which is hard to fix in an appropriate region, but also solve the drawback of the traditional pant-type disposable diaper, which is inconvenient to wear. In using the present invention, the disposable diaper is disposed under the hips, and the removable adhesive strips of the attachable waistband are stuck onto the waistband attachments of the disposable diaper, and then the hook-and-loop fasteners at two ends of the attachable waistband are fastened to each other. Therefore, the present invention is easy to wear without taking off the whole disposable diaper.
2. The present invention features high economic efficiency: the attachable waistband can be repeatedly used for 10-20 times. Therefore, the present invention saves user much money and favors environmental protection.

### BRIEF DESCRIPTION OF THE DRAWINGS

Fig.1 is a diagram schematically showing a disposable diaper according to one embodiment of the present invention; and
Fig.2 is a diagram schematically showing an attachable waistband according to one embodiment of the present invention.

### DETAILED DESCRIPTION OF THE INVENTION

Below, embodiments of the present invention will be described in detail to demonstrate the present invention and make easily understood the objectives and advantages of the present invention. However, it should be understood: these embodiments are only to exemplify the present invention but not to limit the scope of the present invention.

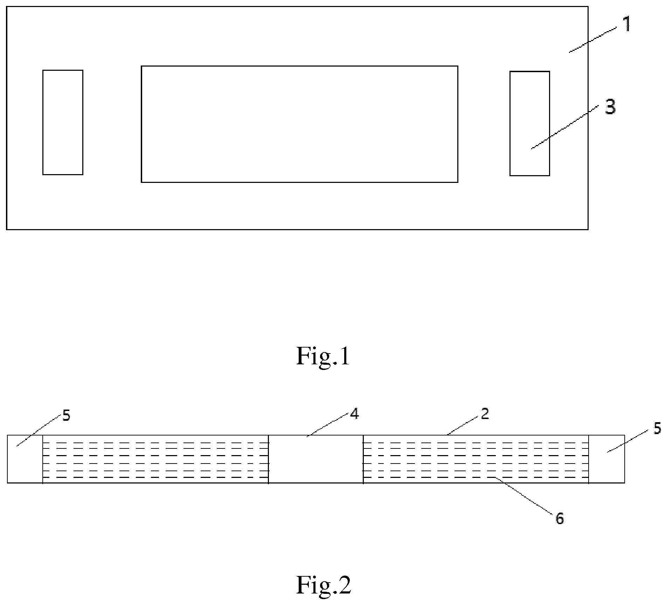

Refer to Fig.1 and Fig.2. In one embodiment, the present invention proposes a disposable diaper with a reusable waistband, which comprises a disposable diaper 1 and an attachable waistband 2. Two waistband attachments 3 are symmetrically formed in the back of the disposable diaper 1. The attachable waistband 2 is made of a non-woven fabric. Several elastic bands 6 are fixed onto the inner side of the attachable waistband 2. Two removable adhesive strips 4 are formed in the middle region of the inner side of the attachable waistband 2 and corresponding to the waistband attachments 3 of the disposable diaper 1. Two corresponding hook-and-loop fasteners 5 are respectively formed on two ends of the attachable waistband 2.

In one embodiment, the girth of the disposable diaper 1 for adults has three sizes: 155-165cm for a large size, 165-175cm for an enlarged size, and 175-190cm for an extra-large size; the girth of the disposable diaper 1 for babies has three sizes: 60-80cm for a large size, 80-100cm for an enlarged size, and 95-115cm for an extra-large size.

In one embodiment, the disposable diaper 1 for babies includes an air-permeable film (originally in dimensions of 320cmx32cm), which is disposed on the bottom layer and has four sizes: 39cmx32cm for a small size, 45cmx32cm for a middle size, 49cmx32cm for a large size, and 54cmx32cm for an enlarged size, wherein the length has a tolerance of 3-5cm, and the width has a tolerance of 1-2cm.

In one embodiment, the hook-and-loop fastener 5 is 30-50cm in length and 20-35cm in width.

In one embodiment, the attachable waistband 2 has 6-18 pieces of elastic bands 6, which are fixed onto the inner side of the attachable waistband 2 with an adhesive glue. The elastic bands 6 provide superior elasticity for the attachable waistband 2. Thereby, the attachable waistband 2 can flexibly contract or extend according to the girth of the user and provides comfort for users and convenience for nursing personnel.

In one embodiment, the attachable waistband 2 is 20-80cm in width. The disposable diaper 1 includes an external water-proof layer, a core layer and an inner cotton layer. The core layer is interposed between the external water-proof layer and the inner cotton layer and disposed between the two waistband attachments 3. The core layer is made of wood pulp and a polymer material.

In using the present invention, the disposable diaper 1 is disposed under the hips, and the removable adhesive strips 4 of the attachable waistband 2 are stuck onto the waistband attachments 3 of the disposable diaper 1, and then the hook-and-loop fasteners 5 at two ends of the attachable waistband 2 are fastened to each other. Thus, the present invention can be put on the user easily. The attachable waistband 2 can be repeatedly used for 10-20 times.

These embodiments described above are only the preferred ones of the present invention. The persons having ordinary knowledge of the art should be able to modify or vary these embodiments without departing from the spirit of the present invention. Any modification or variation according to the spirit of the present invention is to be also included by the scope of the present invention.

## Claims

1. A disposable diaper with a reusable waistband, comprising a disposable diaper (1) and an attachable waistband (2), wherein two waistband attachments (3) are symmetrically formed in a back of said disposable diaper (1); said attachable waistband (2) is made of a non-woven fabric; a plurality of elastic bands (6) is fixed onto an inner side of said attachable waistband (2); two removable adhesive strips (4) are formed in a middle region of said inner side of said attachable waistband (2) and corresponding to said waistband attachments (3); two corresponding hook-and-loop fasteners (5) are respectively formed on two ends of said attachable waistband (2).

2. The disposable diaper with a reusable waistband according to claim 1, wherein a girth of said disposable diaper (1) for adults has three sizes: 155-165cm for a large size, 165-175cm for an enlarged size, and 175-190cm for an extra-large size; a girth of said disposable diaper (1) for babies has three sizes: 60-80cm for a large size, 80-100cm for an enlarged size, and 95-115cm for an extra-large size.

3. The disposable diaper with a reusable waistband according to claim 1, wherein said hook-and-loop fastener (5) is 30-50cm in length and 20-35cm in width.

4. The disposable diaper with a reusable waistband according to claim 1, wherein said attachable waistband (2) has 6-18 pieces of elastic bands (6), which are fixed onto said inner side of said attachable waistband (2) with an adhesive glue.

5. The disposable diaper with a reusable waistband according to claim 1, wherein said attachable waistband (2) is 20-35cm in width.

6. The disposable diaper with a reusable waistband according to claim 1, wherein said disposable diaper (1) includes an external water-proof layer, a core layer and an inner cotton layer; said core layer is interposed between said external water-proof layer and said inner cotton layer and disposed between said two waistband attachments (3).

7. The disposable diaper with a reusable waistband according to claim 6, wherein said core layer is made of wood pulp and a polymer material.
